# FASCICULE DE BREVET EUROPEEN

(11) **EP 3 308 806 B1**
(45) Date de publication et mention de la délivrance du brevet: **12.11.2025**
(21) Numéro de dépôt: 17194165.1
(22) Date de dépôt: 29.09.2017
(51) Int. Cl.: A61L 2/28, G01K 1/20

(54) **DISPOSITIF DE SIMULATION DE CHARGE DE REFERENCE POUR STERILISATEURS A VAPEUR D'EAU**
VORRICHTUNG ZUR SIMULATION DER REFERENZBELADUNG FÜR DAMPFSTERILISATOREN
DEVICE FOR SIMULATING A REFERENCE LOAD FOR STEAM STERILISERS

(30) Priorité: 12.10.2016 FR 1670600
(43) Date de publication de la demande: 18.04.2018
(73) Titulaire: Logiqal, 33700 Merignac (FR)
(72) Inventeur: MORETTI, Eric, 33700 MERIGNAC (FR)
(74) Mandataire: Aquinov

(56) Documents cités:
- EP-A1- 0 762 902
- EP-A1- 1 850 102
- EP-B1- 1 820 519
- WO-A1-2014/046998
- WO-A2-2016/190733

## Description

La présente invention se rapporte à la qualification de performance des stérilisateurs à vapeur d'eau, et en particulier à un dispositif de simulation de charge de référence à cet effet.

Les stérilisateurs à vapeur d'eau ont pour fonction d'inactiver les contaminants microbiologiques présents dans la charge à stériliser pour transformer cette charge non stérile en charge stérile. Il existe des grands stérilisateurs (volume de la chambre supérieur à 60 litres), plutôt destinés aux établissements de soins et aux laboratoires pharmaceutiques, et des petits stérilisateurs (volume de la chambre inférieur à 60 litres) qui sont utilisés dans les cabinets de soins, comme les cabinets dentaires ou les cabinets de gynécologie, ou dans tout autre établissement nécessitant l'utilisation de produits stériles comme les salons de tatouages. Ils fonctionnent tous selon le même principe de vapeur d'eau saturée.

Pour assurer la fonction des stérilisateurs, les utilisateurs doivent porter une attention particulière à la maîtrise du procédé de stérilisation c'est-à-dire la mise au point, la validation et le contrôle de routine du procédé. Cette maîtrise comprend un certain nombre d'activités distinctes mais interdépendantes, comme l'étalonnage, la maintenance, la définition du produit, la définition du procédé, la qualification de l'installation, la qualification opérationnelle et la qualification de performance. On connaît par exemple les documents EP1850102 A1 et EP0762902 A1, qui présentent des appareils pour vérifier l'efficacité d'un procédé de stérilisation à vapeur.

La qualification de performance, régie par un référentiel normatif (NF EN ISO17665), est un processus d'obtention de preuves documentées selon lesquelles le stérilisateur, tel qu'il a été installé, est en mesure de fonctionner de manière reproductible et conformément à des critères prédéterminés, sur une charge de référence et par conséquent de délivrer une charge stérile. La charge de référence comprend des produits qui sont appelés à être stérilisés régulièrement, et qui représentent les familles de produits présentant la plus grande difficulté pour le procédé de stérilisation.

La qualification de performance doit être réalisée régulièrement (généralement annuellement ou bi-annuellement). Actuellement elle est mise en œuvre de la façon suivante : un technicien de mesure place plusieurs enregistreurs de température et pression au cœur d'une charge de stérilisation qui est la charge de référence. Cette charge de référence est constituée de telle sorte qu'elle soit la plus difficile à stériliser. En pratique, on place dans le stérilisateur les charges couramment stérilisées par l'établissement et des enregistreurs de pression et de température, mais il n'existe pas de charge de référence fixe ou normée. Le technicien de mesure effectue un cycle de stérilisation, puis retire les enregistreurs de pression et de température, lit les enregistrements, les analyse, puis reprogramme les enregistreurs pour un deuxième cycle, puis un troisième. La durée d'un cycle de stérilisation est de l'ordre de 60 à 90 minutes si bien que l'enchainement de 3 cycles de stérilisation, auxquels viennent s'ajouter des vérifications techniques complémentaires, prend généralement 5 à 6 heures, soit pratiquement une journée de travail. L'ensemble des données est compilé dans un rapport et vérifié par une personne compétente autre que le technicien de mesure.

Cette procédure est lourde, et donc onéreuse puisqu'elle immobilise un technicien toute la journée.

La conséquence est que seuls les hôpitaux et les cliniques réalisent cette qualification de performance pour les grands stérilisateurs, mais les utilisateurs des petits stérilisateurs, tels que les cabinets libéraux, ne réalisent pas (ou très peu) cette vérification pourtant obligatoire. L'objectif de l'invention est de trouver une solution permettant aux utilisateurs de petits stérilisateurs à vapeur de réaliser une qualification de performance moins onéreuse et aussi fiable que si elle était réalisée avec des charges de routine, par un procédé facile à mettre en œuvre et reproductible.

Pour y répondre, l'invention propose de fournir une charge de référence particulière, plus difficile à stériliser que toutes celles trouvées actuellement chez les utilisateurs de petits stérilisateurs à vapeur d'eau. En particulier l'invention vise un dispositif de simulation de charge de référence pour stérilisateurs à vapeur d'eau, comprenant un conteneur constitué par un fond et un couvercle non étanche, ledit conteneur comprenant :
- au moins un élément de masse présentant un volume d'au moins 40 cm³ et présentant une masse volumique supérieure ou égale à 7 g.cm⁻³,
- au moins un corps creux,
- au moins un enregistreur de température protégé par une barrière stérile,
- au moins un enregistreur de température non protégé par une barrière stérile,
- au moins un enregistreur de pression non protégé par une barrière stérile.

Avantageusement, l'invention permet à la fois de simuler une charge de référence, plus difficile à stériliser qu'une charge de référence composée de produits susceptibles d'être stérilisés régulièrement par l'utilisateur du stérilisateur, et d'enregistrer au cœur de cette charge, les paramètres physiques de pression et de température tout au long du procédé de stérilisation. D'une qualification à l'autre les résultats sont reproductibles car les conditions de charge sont identiques.

Lors de l'enregistrement, l'ensemble formant le dispositif selon l'invention est autonome et ne nécessite aucun accessoire ou système extérieur annexe. Le dispositif 10 selon l'invention peut donc être utilisé directement par l'utilisateur du stérilisateur, qui le place à l'intérieur, l'expose à au moins un cycle, et le fait ensuite analyser.

L'invention a donc également pour objet l'utilisation d'un dispositif 10, pour vérifier la conformité d'un stérilisateur à vapeur d'eau par rapport aux normes attachées audit stérilisateur à vapeur d'eau.

D'autres caractéristiques et avantages de l'invention ressortiront de la description en détail qui va suivre, en regard des dessins annexés sur lesquels :
- la Figure 1 représente une vue en perspective éclatée d'une charge de référence, dans un conteneur,
- la Figure 2 représente une vue de dessous du couvercle dudit conteneur.

L'invention a donc pour objet un dispositif 10 de simulation de charge de référence pour stérilisateurs à vapeur d'eau.

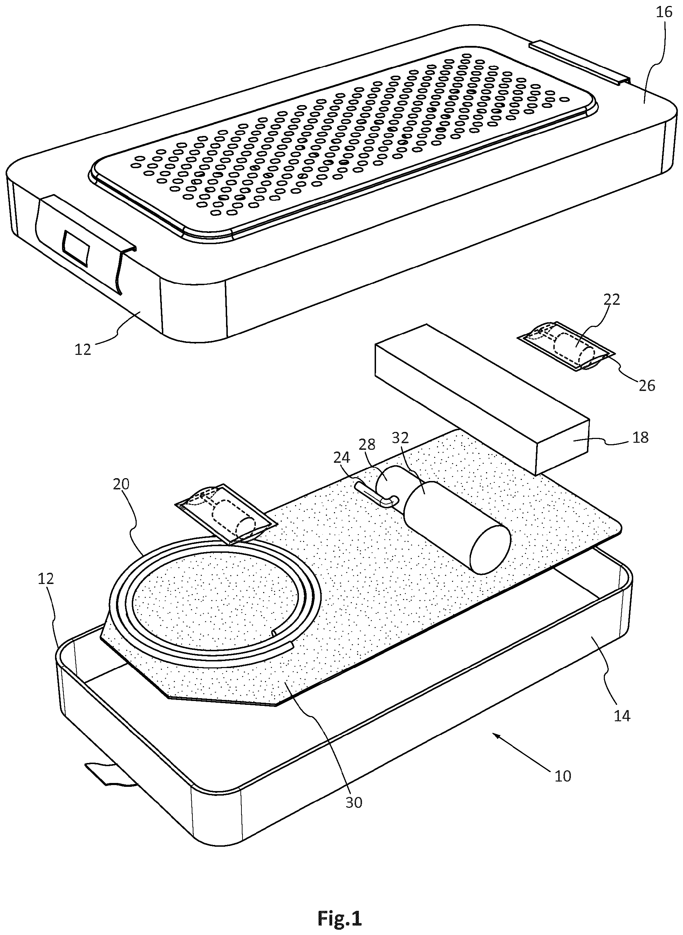

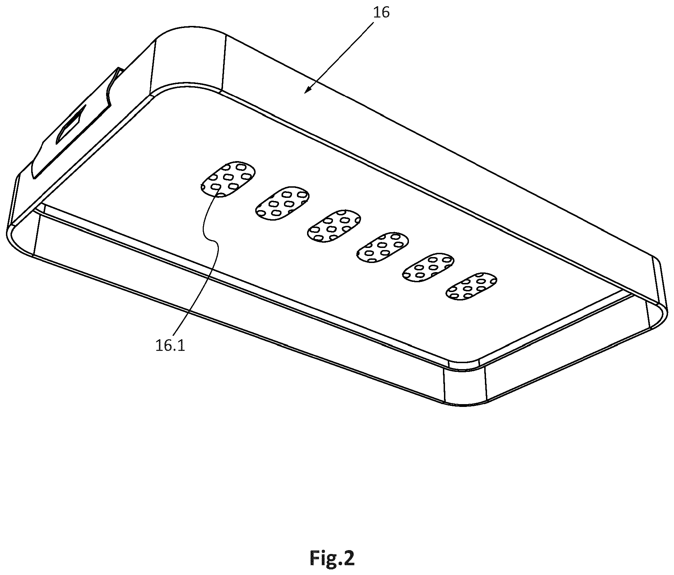

Ainsi que cela est représenté sur la figure 1, le dispositif 10 est constitué par un conteneur 12, qui est une enveloppe de protection mécanique des éléments constituant la charge. Il est constitué par un fond 14 et un couvercle 16 non étanche. Préférentiellement le couvercle 16 comporte au moins une ouverture 16-1 pour la pénétration de la vapeur dans le conteneur lorsqu'il est utilisé dans un stérilisateur, comme montré sur la figure 2.

Le conteneur 12 peut notamment avoir une forme parallélépipédique, avec des dimensions adaptées au stérilisateur à vapeur pour lequel il est destiné.

Le conteneur 12 comprend préférentiellement des moyens de fermeture. De façon préféré, il doit pouvoir être fermé de façon inviolable, et peut donc comporter des moyens de fermeture inviolables comme par exemples des vis de sécurité inviolable.

Le conteneur 12 comprend plusieurs éléments qui concourent ensemble à constituer une charge de stérilisation difficile à stériliser, dont des enregistreurs de température et de pression. Pour créer cette difficulté de stérilisation, le dispositif selon l'invention conjugue plusieurs paramètres :
- hétérogénéité de répartition des masses,
- présence d'au moins un corps creux,
- et préférentiellement hétérogénéité des matériaux utilisés.

En effet, le conteneur 12 comprend :
- au moins un élément de masse 18 présentant une masse volumique supérieure ou égale à 7 g.cm⁻³,
- au moins un corps creux 20,
- au moins un enregistreur de température 22 protégé par une barrière stérile,
- au moins un enregistreur de température 24 non protégé par une barrière stérile 26,
- au moins un enregistreur de pression 28 non protégé par une barrière stérile.

L'élément de masse 18 permet de créer un déséquilibre de la répartition des masses dans le stérilisateur qui provoque des écarts de température dans la chambre du stérilisateur.

L'élément de masse présente un volume d'au moins 40 cm³, plus préférentiellement d'au moins 100 cm³.

Sa masse volumique est supérieure ou égale à 7 g.cm⁻³. Il s'agit préférentiellement d'un élément de masse en acier inoxydable, dont la masse volumique est de 7,98 g.cm⁻³.

Le corps creux 20 joue également un rôle essentiel. En effet, la vapeur d'eau contenue dans la chambre d'un stérilisateur doit être la plus proche possible de la vapeur saturée. Pour atteindre cet objectif, le stérilisateur enchaîne un ou plusieurs cycles d'extraction d'air puis d'injection de vapeur. La présence du corps creux 20 rend l'extraction d'air difficile. Ainsi, si après les cycles d'extraction d'air / injection de vapeur, il reste de l'air dans la chambre du stérilisateur, cela implique que la vapeur n'est pas saturée et que l'efficacité de la stérilisation n'est plus assurée.

Le corps creux 20 peut être de toute forme. Préférentiellement le corps creux présente un volume interne supérieur à 1,5 cm³. La surface de l'ouverture du corps creux 20 est de façon préférée d'au moins 0,75 mm².

Selon une variante, le corps creux 20 est un tube obturé à l'une de ses extrémités. Préférentiellement ledit tube obturé présente au moins l'une des caractéristiques suivantes :
- un diamètre interne d'au moins 1mm,
- une longueur d'au moins 1, 5m.

Le corps creux 20 peut être réalisé en tout matériau, par exemple PTFE.

Selon un mode de réalisation particulièrement adapté, le matériau constituant l'élément de masse 18 et le matériau constituant le corps creux 20 sont différents, créant ainsi une hétérogénéité des matériaux utilisés. Le déséquilibre de comportement de chaque matériau lors des transferts thermiques provoque des écarts de température dans la chambre du stérilisateur.

Pour accentuer cette hétérogénéité, le conteneur 12 peut comprendre un élément supplémentaire constitué en un matériau présentant une conductivité thermique inférieure à une valeur de 0,5 W.m⁻¹.K⁻¹. Il peut s'agir d'un élément en silicone, dont la conductivité thermique est de 0,22 W.m⁻¹.K⁻¹. Le volume de cet élément est préférentiellement supérieur ou égal 50 cm³, encore plus préférentiellement supérieur ou égal à 100 cm³. Il peut s'agir par exemple d'un tapis à picots 30, qui peut être disposé par exemple dans le fond 14 du conteneur sous les autres éléments contenus dans le conteneur 12.

Le conteneur 12 comprend également des enregistreurs :
- au moins un enregistreur de température 22 protégé par une barrière stérile 26,
- au moins un enregistreur de température 24 non protégé par une barrière stérile,
- au moins un enregistreur de pression 28 non protégé par une barrière stérile.

Les enregistreurs sont des enregistreurs autonomes, c'est-à-dire de type embarqué, alimentés sur batterie. Ils disposent d'une mémoire interne permettant l'enregistrement des paramètres horodatés de pression et/ou de température. La capacité de mémoire des enregistreurs est au moins de 1000 points. Les capteurs de température sont préférentiellement des capteurs Pt100 ou Pt1000.

La période de scrutation des enregistreurs, c'est-à-dire l'intervalle entre deux mesures, en particulier la période de scrutation des enregistreurs de température, est préférentiellement inférieure ou égale à 5 secondes.

Au moins un enregistreur de température 22 est protégé par une barrière stérile 26. Selon une variante, le dispositif 10 comprend au moins deux enregistreurs de température protégés par une barrière stérile et ces enregistreurs sont disposés dans des endroits différents dans le conteneur 12.

La barrière stérile 26 est un emballage minimal utilisée pour la stérilisation de certains instruments, qui empêche la pénétration des micro-organismes et qui permet la présentation aseptique des produits stérilisés au point d'utilisation. Les barrières stériles sont utilisées pour protéger les instruments stérilisés à la sortie du stérilisateur de façon à ce qu'il conserve leur caractère stérile pendant plusieurs semaines.

Cette barrière stérile offre une certaine résistance à la pénétration de la vapeur d'eau, donc aux calories qu'elle véhicule. C'est pourquoi, selon l'invention, il est nécessaire de mesurer la température « au cœur de la charge », c'est-à-dire derrière cette barrière stérile.

L'enregistreur ainsi exposé se trouve dans les conditions réelles de stérilisation de produits emballés. La barrière stérile 26 peut être notamment un sachet pelable souple ou un conteneur étanche équipé de filtres constituant une barrière microbienne ou un conteneur étanche équipé de soupapes ou un système de pliage constitué de papier assurant une barrière microbienne. En pratique, il peut s'agir notamment d'un :
- sachet ou gaine dont l'une des faces est en plastique transparent, l'autre en papier poreux à la vapeur,
- papier poreux à la vapeur qui est plié d'une façon spécifique,
- conteneur étanche équipé de filtres en papier poreux à la vapeur,
- conteneur étanche équipé de soupapes .

En plus du ou des enregistreurs de température 22 protégés par une barrière stérile 26, le dispositif 10 selon l'invention comprend également au moins un enregistreur de température 24 et au moins un enregistreur de pression 28, non protégés par une barrière stérile, dans l'ambiance, c'est-à-dire directement en contact avec la chambre du stérilisateur.

L'enregistreur de température 24 et l'enregistreur de pression 28 non protégés par une barrière stérile peuvent être regroupés en un seul dispositif 32 capable d'enregistrer à la fois la température et la pression. Un tel dispositif 32 permet l'enregistrement de plusieurs paramètres simultanément, horodatés ensemble.

Le conteneur peut également comprendre d'autres éléments.

Selon une variante un ou plusieurs enregistreurs pourraient être placés en dehors du conteneur 12, ce qui donnerait des résultats équivalents.

Le montage du dispositif 10 selon l'invention est réalisé en disposant l'ensemble des constituants dans le conteneur 12. Préférentiellement au moins un enregistreur de température 22 protégé par une barrière stérile est disposé en périphérie dans le conteneur. De même, un dispositif préféré consiste à disposer l'élément de masse 18 et le corps creux 20 de façon opposée dans le conteneur. Préférentiellement l'enregistreur de température 24 et l'enregistreur de pression 28 ou l'enregistreur de température et pression 32 est disposé approximativement au centre du conteneur 12.

Le dispositif 10 peut être incorporé dans une valise dédiée pour faciliter son transport entre l'utilisateur du stérilisateur et le centre qui analysera les données relatives à la performance du stérilisateur.

Le dispositif 10 selon l'invention est donc destiné à être utilisé pour la qualification de performance de stérilisateurs à vapeur d'eau, en particulier de stérilisateurs à vapeur d'eau dont le volume de la chambre de stérilisation est inférieur à 60 litres, c'est-à-dire pour vérifier la conformité d'un stérilisateur à vapeur d'eau par rapport aux normes qui lui sont attachées, telles que la norme NF EN ISO17665.

Pour son utilisation, le dispositif est disposé dans la chambre de stérilisation, puis soumis à au moins un cycle de stérilisation, préférentiellement au moins trois cycles.

Selon un mode de réalisation particulièrement adapté, le dispositif 10 est utilisé en mettant en œuvre les étapes suivantes :
- Disposer le dispositif 10 dans la chambre de stérilisation du stérilisateur,
- Appliquer au moins un cycle dont la phase de stérilisation s'établit à une température comprise entre 120°C et 140°C pendant une durée maximale de 30 minutes, préférentiellement au moins trois cycles,
- Récupérer le dispositif 10,
- Analyser les données enregistrées par les enregistreurs de température et pression pour vérifier la conformité du stérilisateur aux normes attachées audit stérilisateur à vapeur d'eau.

L'application des cycles de stérilisation peut être réalisée comme suit :
- Cycle test de pénétration vapeur pendant 2 à 5 minutes à une température comprise entre 120°C et 140°C,
- 3 cycles successifs de stérilisation, chacun pendant 15 à 20 minutes à une température comprise entre 120°C et 140°C.

En plus du dispositif 10 selon l'invention, il est possible de placer avant le cycle test de pénétration vapeur un test Helix dans la chambre du stérilisateur et de le récupérer après ce cycle.

Avant utilisation du dispositif 10, il convient de programmer les enregistreurs, d'insérer le ou les enregistreur(s) de température 22 derrière une barrière stérile 26 et de fermer le couvercle 16. Cette opération est préférentiellement réalisée par un professionnel ; elle peut être mise en œuvre plusieurs jours avant l'utilisation. Une fois préparé, le dispositif 10 peut être envoyé à l'utilisateur du stérilisateur, par exemple dans une valise.

Après réception, l'utilisateur place le dispositif 10 dans le stérilisateur et met en œuvre les étapes telles que décrites ci-avant.

Après utilisation, le dispositif est préférentiellement transmis à une personne habilitée à analyser la performance du stérilisateur. Le couvercle 16 du conteneur 12 est ouvert, et les enregistreurs sont récupérés pour lire les données qu'ils contiennent. Les données sont ensuite traitées numériquement, comparées aux valeurs de référence attachées au stérilisateur selon la norme en vigueur, et un rapport de mesures est établi déterminant si le stérilisateur est conforme ou non conforme.

De même, il est préférable de récupérer les enregistrements des cycles mis en œuvre lors de l'utilisation du dispositif, de façon à contrôler que le bon procédé de stérilisation a été réalisé pour le test de qualification de performance.

Après utilisation, le dispositif 10 peut être réutilisé. Il convient cependant de programmer de nouveau les enregistreurs, d'insérer le ou les enregistreur(s) de température 22 derrière une barrière stérile 26 et de fermer le couvercle 16.

## Revendications

1. Dispositif (10) de simulation de charge de référence pour stérilisateurs à vapeur d'eau, constitué par un conteneur (12) comprenant un fond (14) et un couvercle (16) non étanche, ledit conteneur (12) incluant :
- au moins un élément de masse (18) présentant un volume d'au moins 40 cm³ et présentant une masse volumique supérieure ou égale à 7 g.cm⁻³,
- au moins un corps creux (20),
- au moins un enregistreur de température (22) protégé par une barrière stérile (26),
- au moins un enregistreur de température (24) non protégé par une barrière stérile,
- au moins un enregistreur de pression (28) non protégé par une barrière stérile.

2. Dispositif (10) selon l'une des précédentes revendications, **caractérisé en ce que** l'élément de masse (18) est en acier inoxydable.

3. Dispositif (10) selon l'une des précédentes revendications, **caractérisé en ce que** le corps creux (20) présente un volume interne supérieur à 1,5 cm³.

4. Dispositif (10) selon l'une des précédentes revendications, **caractérisé en ce que** la surface de l'ouverture du corps creux (20) est d'au moins 0,75 mm².

5. Dispositif (10) selon l'une des précédentes revendications, **caractérisé en ce que** le corps creux (20) est un tube obturé à l'une de ses extrémités.

6. Dispositif (10) selon la précédente revendication, **caractérisé en ce que** le tube présente un diamètre interne d'au moins 1mm.

7. Dispositif (10) selon l'une des revendications 5 ou 6, **caractérisé en ce que** le tube présente une longueur d'au moins 1, 5m.

8. Dispositif (10) selon l'une des précédentes revendications, **caractérisé en ce que** le matériau constituant l'élément de masse (18) et le matériau constituant le corps creux (20) sont différents.

9. Dispositif (10) selon l'une des précédentes revendications, **caractérisé en ce que** le conteneur (12) comprend un élément supplémentaire constitué en un matériau présentant une conductivité inférieure à 0,5 W.m⁻¹.K⁻¹.

10. Dispositif (10) selon l'une des précédentes revendications, **caractérisé en ce que** le conteneur comprend un tapis à picots (30).

11. Dispositif (10) selon l'une des précédentes revendications, **caractérisé en ce que** les enregistreurs de température (22, 24) présentent une période de scrutation inférieure ou égale à 5 secondes.

12. Dispositif (10) selon l'une des précédentes revendications, **caractérisé en ce que** l'enregistreur de pression (28) et l'enregistreur de température (24) non protégé par une barrière stérile sont regroupés en un seul dispositif (32) capable d'enregistrer à la fois la pression et la température.

13. Dispositif (10) selon l'une des précédentes revendications, **caractérisé en ce qu'**il comprend au moins deux enregistreurs de température (22) protégés par une barrière stérile.

14. Dispositif (10) selon l'une des précédentes revendications, **caractérisée en ce que** le système de barrière stérile (26) est un sachet pelable souple ou un conteneur étanche équipé de filtres constituant une barrière microbienne ou un conteneur étanche équipé de soupapes ou un système de pliage constitué de papier assurant une barrière microbienne.

15. Utilisation d'un dispositif selon l'une des revendications 1 à 14, pour vérifier la conformité d'un stérilisateur à vapeur d'eau par rapport aux normes attachées audit stérilisateur à vapeur d'eau.

16. Utilisation selon la revendication 15, **caractérisée en ce que** le stérilisateur à vapeur d'eau dont le volume de la chambre de stérilisation est inférieur à 60 litres.

17. Utilisation selon la revendication 15 ou 16, **caractérisée en ce que** le dispositif (10) est disposé dans la chambre de stérilisation du stérilisateur puis soumise à au moins un cycle de stérilisation.

18. Utilisation selon l'une des revendications 15 à 17, **caractérisée en ce qu'**elle comprend les étapes suivantes :
- Disposer le dispositif (10) dans la chambre de stérilisation du stérilisateur,
- Appliquer au moins un cycle dont la phase de stérilisation s'établie à une température comprise entre 120°C et 140°C pendant une durée maximale de 30 minutes,
- Récupérer le dispositif (10),
- Analyser les données enregistrées par les enregistreurs pour vérifier la conformité du stérilisateur aux normes attachées audit stérilisateur à vapeur d'eau.

## Patentansprüche

1. Vorrichtung (10) zum Simulieren einer Referenzbelastung für Wasserdampfsterilisatoren, bestehend aus einem Behälter (12), umfassend einen Boden (14) und einen undichten Deckel (16), wobei der Behälter (12) einschließt:
- mindestens ein Masseelement (18), das ein Volumen von mindestens 40 cm³ und eine Dichte größer als oder gleich 7 g.cm⁻³ aufweist,
- mindestens einen Hohlkörper (20),
- mindestens einen Temperaturschreiber (22), der durch eine sterile Barriere (26) geschützt ist,
- mindestens einen Temperaturschreiber (24), der nicht durch eine sterile Barriere geschützt ist,
- mindestens einen Druckschreiber (28), der nicht durch eine sterile Barriere geschützt ist.

2. Vorrichtung (10) nach einem der vorstehenden Ansprüche,
**dadurch gekennzeichnet, dass** das Masseelement (18) aus Edelstahl ist.

3. Vorrichtung (10) nach einem der vorstehenden Ansprüche,
**dadurch gekennzeichnet, dass** der Hohlkörper (20) ein Innenvolumen von mehr als 1,5 cm³ aufweist.

4. Vorrichtung (10) nach einem der vorstehenden Ansprüche,
**dadurch gekennzeichnet, dass** die Oberfläche der Öffnung des Hohlkörpers (20) mindestens 0,75 mm² ist.

5. Vorrichtung (10) nach einem der vorstehenden Ansprüche,
**dadurch gekennzeichnet, dass** der Hohlkörper (20) ein Rohr ist, das an einem seiner Enden geschlossen ist.

6. Vorrichtung (10) nach dem vorstehenden Anspruch,
**dadurch gekennzeichnet, dass** das Rohr einen Innendurchmesser von mindestens 1 mm aufweist.

7. Vorrichtung (10) nach einem der Ansprüche 5 oder 6,
**dadurch gekennzeichnet, dass** das Rohr eine Länge von mindestens 1,5 m aufweist.

8. Vorrichtung (10) nach einem der vorstehenden Ansprüche,
**dadurch gekennzeichnet, dass** das Material des Masseelements (18) und das Material des Hohlkörpers (20) unterschiedlich sind.

9. Vorrichtung (10) nach einem der vorstehenden Ansprüche,
**dadurch gekennzeichnet, dass** der Behälter (12) ein zusätzliches Element umfasst, das aus einem Material besteht, das eine Leitfähigkeit von weniger als 0,5 Wm-¹.K⁻¹ aufweist.

10. Vorrichtung (10) nach einem der vorstehenden Ansprüche,
**dadurch gekennzeichnet, dass** der Behälter eine Stachelmatte (30) umfasst.

11. Vorrichtung (10) nach einem der vorstehenden Ansprüche,
**dadurch gekennzeichnet, dass** die Temperaturschreiber (22, 24) eine Abtastperiode von kleiner als oder gleich 5 Sekunden aufweisen.

12. Vorrichtung (10) nach einem der vorstehenden Ansprüche,
**dadurch gekennzeichnet, dass** der Druckschreiber (28) und der Temperaturschreiber (24), die nicht durch eine sterile Barriere geschützt sind, in einer einzigen Vorrichtung (32) zusammengefasst sind, die in der Lage ist, sowohl Druck als auch Temperatur festzuschreiben.

13. Vorrichtung (10) nach einem der vorstehenden Ansprüche,
**dadurch gekennzeichnet, dass** sie mindestens zwei Temperaturschreiber (22) umfasst, die durch eine sterile Barriere geschützt sind.

14. Vorrichtung (10) nach einem der vorstehenden Ansprüche,
**dadurch gekennzeichnet, dass** das System der sterilen Barriere (26) ein flexibler abziehbarer Beutel oder ein dichter Behälter, der mit Filtern ausgestattet ist, die eine mikrobielle Barriere bilden, oder ein dichter Behälter, der mit Ventilen ausgestattet ist, oder ein Faltsystem aus Papier ist, das eine mikrobielle Barriere sicherstellt.

15. Verwendung einer Vorrichtung nach einem der Ansprüche 1 bis 14 zum Überprüfen der Konformität eines Wasserdampfsterilisators hinsichtlich der Normen, die für den Wasserdampfsterilisator gelten.

16. Verwendung nach Anspruch 15, **dadurch gekennzeichnet, dass** der Wasserdampfsterilisator, dessen Sterilisationskammervolumen weniger als 60 Liter beträgt.

17. Verwendung nach Anspruch 15 oder 16, **dadurch gekennzeichnet, dass** die Vorrichtung (10) in die Sterilisationskammer des Sterilisators eingebracht und anschließend mindestens einem Sterilisationszyklus unterzogen wird.

18. Verwendung nach einem der Ansprüche 15 bis 17,
**dadurch gekennzeichnet, dass** sie die folgenden Schritte umfasst:
- Einbringen der Vorrichtung (10) in die Sterilisationskammer des Sterilisators,
- Anwenden mindestens eines Zyklus, dessen Sterilisationsphase bei einer Temperatur zwischen 120 °C und 140 °C für eine maximale Dauer von 30 Minuten durchgeführt wird,
- Zurückholen der Vorrichtung (10),
- Analysieren der Daten, die durch die Schreiber festgeschrieben wurden, zum Überprüfen der Konformität des Sterilisators mit den Normen, die für den Wasserdampfsterilisator gelten.

## Claims

1. Reference load simulation device (10) for steam sterilizers, consisting of a container (12) comprising a bottom (14) and a non-sealed lid (16), said container (12) including:
- at least one mass element (18) having a volume of at least 40 cm³ and a density greater than or equal to 7 g.cm⁻³,
- at least one hollow body (20),
- at least one temperature recorder (22) protected by a sterile barrier (26),
- at least one temperature recorder (24) not protected by a sterile barrier,
- at least one pressure recorder (28) not protected by a sterile barrier.

2. Device (10) according to one of the preceding claims,
**characterized in that** the mass element (18) is made of stainless steel.

3. Device (10) according to either of the preceding claims,
**characterized in that** the hollow body (20) has an internal volume greater than 1.5 cm³.

4. Device (10) according to any of the preceding claims, **characterized in that** the surface area of the opening of the hollow body (20) is at least 0.75 mm².

5. Device (10) according to any of the preceding claims,
**characterized in that** the hollow body (20) is a tube closed at one of its ends.

6. Device (10) according to the preceding claim, **characterized in that** the tube has an internal diameter of at least 1 mm.

7. Device (10) according to one of claims 5 or 6, **characterized in that** the tube has a length of at least 1.5 m.

8. Device (10) according to any of the preceding claims,
**characterized in that** the material constituting the mass element (18) and the material constituting the hollow body (20) are different.

9. Device (10) according to any of the preceding claims,
**characterized in that** the container (12) comprises an additional element made of a material having a conductivity of less than 0.5 W.m⁻¹.K⁻¹.

10. Device (10) according to any of the preceding claims,
**characterized in that** the container comprises a spiky mat (30).

11. Device (10) according to any of the preceding claims,
**characterized in that** the temperature recorders (22, 24) have a scanning period less than or equal to 5 seconds.

12. Device (10) according to any of the preceding claims,
**characterized in that** the pressure recorder (28) and the temperature recorder (24) not protected by a sterile barrier are combined into a single device (32) capable of recording both pressure and temperature.

13. Device (10) according to any of the preceding claims, **characterized in that** it comprises at least two temperature recorders (22) protected by a sterile barrier.

14. Device (10) according to any of the preceding claims, **characterized in that** the sterile barrier system (26) is a flexible peelable pouch or a sealed container equipped with filters constituting a microbial barrier or a sealed container equipped with valves or a folding system consisting of paper ensuring a microbial barrier.

15. Use of a device according to any of claims 1 to 14, for checking the compliance of a steam sterilizer with the standards attached to said steam sterilizer.

16. Use according to claim 15, **characterized in that** the steam sterilizer, the sterilization chamber volume of which is less than 60 liters.

17. Use according to claim 15 or 16, **characterized in that** the device (10) is placed in the sterilization chamber of the sterilizer and then subjected to at least one sterilization cycle.

18. Use according to any of claims 15 to 17, **characterized in that** it comprises the following steps:
- Placing the device (10) in the sterilization chamber of the sterilizer,
- Applying at least one cycle with a sterilization phase at a temperature between 120°C and 140°C for a maximum of 30 minutes,
- Retrieving the device (10),
- Analyzing the data recorded by the recorders to check the compliance of the sterilizer with the standards attached to said steam sterilizer.
